# EUROPEAN PATENT APPLICATION

(11) **EP 4 360 672 A1**
(43) Date of publication of application: **01.05.2024**
(21) Application number: 22826647.4
(22) Date of filing: 18.05.2022
(51) Int. Cl.: A61M 5/178, A61M 5/315, B01L 3/00, A61B 5/15, B04B 5/04, B04B 11/04

(54) **SYSTEM FOR TREATING BIOLOGICAL MATERIAL**

(30) Priority: 22.06.2021 ES 202130581
(71) Applicant: Cantero Burgaz, José, 08980 Barcelona (ES)
(72) Inventor: CANTERO BURGAZ, José, 08980 BARCELONA (ES)
(74) Representative: Matamoron Hernandez, José Pedro
(86) International application number: PCT/ES2022/070306
(87) International publication number: WO 2022/269108

(57) **Abstract**

The invention relates to a medical veterinary device for extracting and containing biological material to be processed, wherein the medical veterinary device (100) comprises interchangeable pieces for forming a syringe (110a) for extracting biological material, a container (100b) for the extracted biological material to be processed, and a syringe or system for applying the biological material contained in the container (100b). The invention also relates to equipment for processing a biological material contained in the medical veterinary device.

## Description

### System for the treatment of biological material

### Scope

The present invention refers to a system for the treatment of biological material that comprises a device for the extraction of biological material, such as blood, bone marrow, adipose tissue, semen, synovial fluid or other body liquids and fluids, and equipment for processing the biological material contained in said medical-veterinary device, by means of centrifugation, agitation, filtering, temperature, emulsification or fragmentation.

### Prior state of the art

Some systems used for the processing of biological material are currently known, which, among others, include syringes for the extraction of the referred biological material from the patient, as well as several containers suitable to contain said biological material and to carry out its processing in a suitable machine in order to carry out its processing, centrifugation, agitation, filtering, temperature, emulsification or fragmentation, for example, in order to separate it from the different fractions of whole blood, select them, and then extract them and apply the desired fraction.

A general problem with these systems is that the syringes have suitable characteristics in order to carry out the extraction of the biological material from the patient, making it necessary to transfer said biological material to other containers with different characteristics, and suitable for treating it in a specific centrifugal, filtering, fragmentation, thermal, ultrasonic or agitating machine.

Given the type of biological material to be processed, in order to obtain satisfactory results safely and without risk, it is essential that said material is not contaminated from the moment it is extracted until the end of its processing, due to the fact that the risk of contamination increases while being processed in the number of containers used and transfers from one container to another, as well as inputs and outputs of connections between them.

The technical problem that arises is to minimize the number of containers, devices, transfers and equipment to be used in order to carry out the extraction, processing and subsequent application of the processed biological material or a fraction extracted from it and to optimize the size, time, volume and operation of the processing equipment.

### Explanation of the invention

The system of the invention comprises a medical-veterinary device for the extraction of fluids/liquids and/or biological tissues, for example: blood, adipose tissue, bone marrow, semen, synovial fluid or other fluids and body fluids, in addition to equipment for processing the biological material contained in said medical-veterinary device; featuring the particularity that said medical-veterinary device has technical characteristics that allow, through the use and exchange of a reduced number of parts/components, to alternately form:
- a syringe suitable for aspirating fluids or biological tissues of different densities,
- an airproof, inert, aseptic and sterile container that holds the extracted biological material inside and that is suitable to be introduced into the processing equipment to process that biological material by means of centrifugation / agitation / filtration / temperature / ultrasound / fragmentation, in a very small space and in the volumes of interest, according to the criteria of the user or,
- a syringe for the individualized extraction of the fractions of biological material of interest separated during its processing as well as its subsequent use or application by injection, spraying, mixing with autologous or homologous biomaterials or synthetic and natural substitutes or applying it topically, according to the needs and the criteria of the user.

This container makes it possible to reattach the necessary parts/components to once again convert the veterinary-medical device into a syringe in order to inject its contents or separate fractions of the processed biological material.

The device of the invention comprises a cylindrical, transparent tube, which has a conical front end with a connector, preferably a male Luer Lock type, and an open rear end, both ends being provided with reversible coupling and fixing means for the interchangeable parts that allow its conversion into a syringe or into a container protected by end caps.

The device comprises, inside, a plunger that can be moved inside the cylindrical tube, comprising: - a rigid rear part, with a tubular configuration and a section which is smaller than the interior of the cylindrical tube, and - a front part made of flexible material with a diameter fitted to the rigid tailpiece and the internal diameter of the cylindrical tube, coupled to the rigid, tubular-configured tailpiece. Said front part is deformable between: a convex initial position coinciding and in contact with the conical front end of the cylindrical tube, and a concave position automatically adopted during the suction/aspiration of the biological material to be processed and its possible future application/use in the patient.

The flexible front part of the plunger includes an orifice with a coupled connector in its central area, optionally a valved female Luer Lock cone type, accessible through the rear mouth of the cylindrical tube. In order to avoid adhesions between the rigid material of the cylindrical tube and the flexible material in the flexible front part of the piston, this front part is positively embossed in the form of radially arranged lines or points, integrated in the same flexible material and made of the same material.

Said medical-veterinary device also comprises a first piston actuation rod, provided with a front connector that can be coupled to the female Luer Lock cone-type valved connector, integrated into the component of the flexible part of the piston and whose displacement causes the change of initial convex position to the concave position of use of the flexible front part of the piston during the suction of blood or biological material and its possible injection or subsequent application.

In an embodiment of the invention, the medical-veterinary device comprises a second, transparent stem, with an axial channel, provided with: a male Luer cone type back connector that can be coupled to the female Luer cone type valved connector of the flexible component of the plunger and secured by a bayonet-type fixation to the rigid component of the plunger and provided with a male Luer cone-type tip for actuation of the female Luer Lock cone-type valved connector, and another valved back connector, both connected by a fine axial conduit and extraction/introduction of blood fractions, biological material or liquids externally or contained in the cylindrical processing tube and separated by centrifugation or by suitable processing.

The change of the flexible front part of the piston, from a convex initial position to a concave position of use, provides an essential technical advantage for the invention, since in said initial position it features a shape that coincides with the conical shape of the front end of the tube and is kept in contact with said conical front end of the cylindrical tube on the positive radial reliefs of the flexible component, thus assuring the minimum initial existence of air inside the cylindrical tube, and allowing the desired amount of volume of tissues or fluids to be aspirated without subtracting any volume of fluids due to the presence of air.

The change of position from convex to concave of the flexible front part of the plunger in the position of use, prevents the formation of turbulence during the extraction of the separated fractions of processed biological material through the female Luer Lock cone-type connector of the plunger, and of the second rod with axial channel connected to the female Luer cone connector and secured and fixed to the rigid part of the plunger by means of a bayonet-type thread, and minimizes the risk of mixing of the fractions when performing suction through the central area, more backward, of the concave shape of the flexible front part of the piston. Alternatively, standard syringes attached to the plunger valve can be used in order to carry out fraction withdrawal.

The use of the hollow and transparent cylindrical tube in combination with the rigid part of the transparent plunger and the axial conduit of the transparent rod, for the extraction and separation of the fractions of biological material contained in the cylindrical tube and separated during processing, allows, for example, the visualization of the color of each fraction that is being extracted, and the change in the color indicates how far to continue or stop in order to select the optimum fraction to be used by the user. When the next fraction enters said fine transparent axial conduit, it allows to obtain a great precision in the separate extraction of each fraction and to avoid the accidental extraction of a part of the following fraction, without mixing them and with a full visual control by the user.

Optionally, the detection of a variation in the composition of the extracted fractions can be carried out by optical, digital, impedance, laser or other methods that can make very precise readings of the biological material without mixing them and with fully automatic control.

Advantageously, the rear connector of the second rod with axial channel is a valved connector, for example, a female Luer Lock type, or of another type suitable for the connection of a syringe with a male Luer or Luer Lock cone for the extraction and separation of the fraction of biological material closest to the plunger inside the cylindrical tube, with a syringe, or with an extension tube provided with a suitable connector for the introduction or extraction into/from the interior of the cylindrical tube, through the second rod with axial channel, of a possible auxiliary liquid or other body fluid or tissue through the transparent plunger, that is to say, through the rear part of the plunger coupled to the stem and as desired, without breaking the closed circuit. Both connectors of the device are used in order to withdraw or add fluids, liquids, tissues, fractions or medications, depending on the protocol and the interest of the user.

The cylindrical tube comprises removable closure caps with fixation, at the anterior and posterior ends of the cylindrical tube and which, together with said cylindrical tube and its plunger, make up a container that can be attached to equipment for processing fluids and biological tissues and for obtaining of concentrates or biological products and by-products by differential centrifugation, agitation, filtration, fragmentation, ultrasound or temperature.

Said cylindrical tube also comprises a removable rear stop, provided with: coupling and fixing means with the rear end of the cylindrical tube, lateral support fins for the user's fingers, and a hole for passage and retention in different longitudinal positions of the rods and consequently of the plunger connected to them, for vacuum or removal of fractions.

The aforementioned first and second rods have an ergonomic rear wing for their actuation (in use by pulling or pushing them) and longitudinal wings with longitudinally spaced teeth, which interact with the rear stop with tabs in the different retention positions, for its segmentation in steps of a predetermined volume, for example, in 5 ml steps.

In a first angular position of the first stem, without axial channel, said lateral toothed tabs in segments of predetermined volume pass through the removable abutting stopper hole coupled to the rear end of the cylindrical tube, allowing its longitudinal displacement; while in a second angular position of the first rod, the tabs are hooked in the removable stop, thus preventing the longitudinal movement of said plunger.

This feature makes it possible to move the plunger towards the rear area of the cylindrical tube, keeping the valve at the front end of the cylindrical tube closed, and to fix it to the removable stopper by means of the side tabs, creating a predetermined vacuum inside the cylindrical tube for the suction of the biological material during its extraction.

With the characteristics mentioned, this medical-veterinary device features a series of advantages compared to those currently existing, among which it is worth mentioning, that it is:
- Safe: like a syringe, but it can be disassembled and can be reassembled, always in a closed system.
- Flexible: the same medical-veterinary device is used for any fluid or biological tissue and for any volume between 1ml. and 100 ml. and in any medical, veterinary, or analytical setting (ambulatory, surgical, or laboratory).
- Simple: It is used as a syringe that allows aspiration with a self-vacuum system, if required, it becomes a container to process in centrifugation, temperature, agitation, filtration, fragmentation, ultrasound, laser, grinding, digestion or other processors, in addition to accurately selecting the biological product and finally allowing the concentrate to be injected directly, if required.
- Practical: The same device is at the same time a syringe to extract, a container to process and is once again a syringe to infiltrate or extract the separated fractions of biological material, from small to large volumes (between 1ml. and 100m. per device).
- Easily mountable and removable. It is transformed into a cylindrical tube with 2 outlets and which can be processed in 2 different positions, in almost any processing equipment currently available in the market by centrifugation, filtration, agitation, fractionation, temperature, ultrasound, laser, grinding or digestion which admits tubes with the size of the device and it is also an exclusive device for the automated processing equipment of the invention.

According to the invention, the processing equipment comprises: a microprocessor control structure and electronics, a structural chassis, an outer casing provided with a closing lid with a safety lock, a touchpad, a motor-driven turntable maintenance-free electric bidirectional rotation, and it is provided with at least two fixings for placing the tubes/cylindrical containers of the biological material to be processed and other cylindrical tubes/containers for collecting a separate fraction of the biological material processed.

In order to transfer a fraction of the processed biological material from one container to another container, the processing equipment comprises a transparent flexible tube (for example silicone) with connectors that can be attached to the Luer Lock connectors located at the front ends of the cylindrical tubes and / or of the internal pistons of the respective tubes/cylindrical tanks; and of one or more peristaltic or other pump(s) for the transfer of a volume or a separate fraction of biological material from one container to another, by said flexible tube, and alternatively for any liquid or compound necessary for the different techniques and uses, at the discretion of the user.

This processing equipment also includes a control unit with specific software to work with or without Internet connection, as independent equipment, carrying out predefined processing cycles and to work "on line/off line" interlinked with a counter/analyzer (for example, a hematology analyzer) and to carry out personalized processing communication being suitable for receiving information on data relating to the patient, date, diagnosis, therapy, clinical history, process, initial product to be processed, the handling and/or sending of data related to the final product obtained from the processing and the connection with other equipment, with apps and with the cloud. Statistics, protocols, instructions and other data obtained will make up Big Data in order to provide improvements and optimizations of applications and therapies with equipment, systems and devices for the benefit of patients.

According to the invention, the aforementioned processing equipment comprises at least one support for holding an intermediate portion of the transparent tube, preferably flexible and made of silicone, used to transfer the product from one cylindrical tube to another cylindrical tube and a sensor (optical, digital, by impedance, ultrasonic, by laser or others) that detects changes in color, density, components of the final product transferred through the transparent flexible tube and that causes speed variation, reversal/inversion of flow direction or stoppage of the peristaltic pump(s) when it detects a change (for example in color) of the product circulating through the flexible tube.

Also optionally the sensor in the flexible tube line can be a detector, reader, counter and identifier of particles, their size, their number and their geometry/morphology or biological, chemical or biochemical composition, by means of imaging technology, impedance, laser or other technologies that conform to these readings, counts and identifications.

### Brief description of the content of the drawings

In order to complement the description that is being made and in order to facilitate the understanding of the characteristics of the invention, a set of drawings is attached to this specification in which, for illustrative and nonlimiting purposes, the following has been represented:
- Figure 1 shows a schematic view of an embodiment of the system for processing biological material object of the invention.
- Figure 2 shows an exploded view in elevation of the different components of the medical-veterinary device intended to be coupled alternatively with said cylindrical tube, in order to form a syringe for extracting biological material, a container for processing biological material, or an extraction syringe from the container of the processed biological material fractions and alternatively an injection/application syringe of the biological material and/or its fractions.
- Figure 2a shows a front view of the plunger, in which lines and points in relief can be seen defined in the flexible front part thereof.
- Figures 3 and 4 show perspective views of the medical-veterinary device respectively forming a syringe and a container
- Figure 5 shows a perspective view of the stopper with detachable fins designed to fit onto the rear end of the cylindrical tube in order to form the syringe configuration.
- Figure 6 shows an elevation view, sectioned by a vertical plane of the syringe ready for the extraction of biological material; with the front part of the piston in a convex initial position, in contact with the conical front end of the cylindrical tube; with the suction plunger connected to the first rod, without axial channel, and with a straight needle, mounted on the male Luer Lock cone connector or similar, at the anterior end of the cylindrical tube.
- Figure 7 shows a view of the syringe of figure 6, once an extraction of biological material has been carried out, specifically blood, with the plunger displaced and the flexible part of the plunger modified in a concave position and the plunger assembly moved towards the zone back of the cylindrical tube.
- Figure 8 shows a view of the syringe of Figure 7, once the blood extraction has been carried out, and the first driving rod has been removed, and with the front and rear covers attached to the ends of the tube, forming a container for the processing of biological material in processing equipment. In case of processing the device in the equipment designed to carry out the separations of fractions in an automated way, the stoppers are not necessary.
- Figure 9 shows a profile view of the syringe with the plunger in the position of figure 8, containing the blood fractions obtained by processing, specifically centrifuging in this case. In this figure, the second rod, with an axial channel, is coupled to the valved connector and fixed by means of a bayonet-type closure to the rigid plunger for the extraction of the blood fractions obtained and with a syringe, for the collection of the fraction obtained above the cylindrical tube connected to the rear connector of said second axial channel.
- Figure 10 shows a schematic elevation view of an embodiment of the equipment for processing the biological material contained in the cylindrical tube of Figures 4 and 8, and in which the different elements housed inside the equipment have been represented.
- Figures 11, 12 and 13 show schematic plan views of the processing equipment of the previous Figure during the separation into fractions of a biological sample housed in the cylindrical tube that functions as a container for the medical-veterinary device and the transfer from one of the separated fractions to a second cylindrical tube that also functions as a container.
- Figure 14 shows a plan view of a variant embodiment of the processing equipment working simultaneously with two pairs of cylindrical tubes.
- Figure 15 shows a variant embodiment of the system shown in Figure 1, provided in this case with means of intercommunication for its connection with other external equipment.

### Detailed description of embodiments of the invention

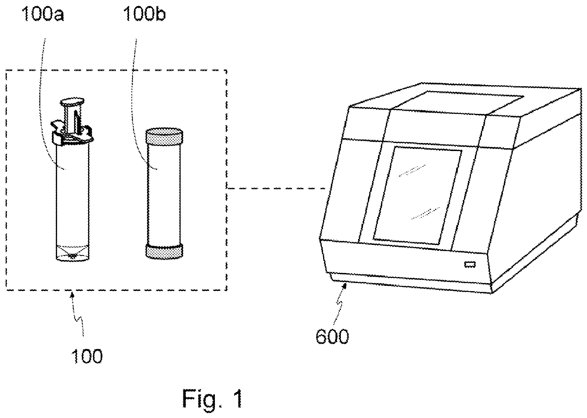

In Figure 1, the medical-veterinary device (100) has been schematically represented, using the format of a syringe (100a) and the format of a container (100b) conformable with the same device, and the appropriate processing equipment (600) in order to process the biological samples inside the container format (100b) of the medical-veterinary device (100).

The medical-veterinary device (100) comprises a cylindrical tube (10) that has a conical front end (101), equipped with a connector (102), in this case a male Luer Lock cone, for coupling a needle, trocar, cannula or tube, depending on the biological material to be processed, (blood, bone marrow, adipose tissue, etc.) provided with a complementary connection and an open rear end (103).

The front and rear ends (101, 103) of the cylindrical tube (10) comprise coupling means (104, 105) for assembling a rear stop (30) at the rear end of the cylindrical tube during use of the medical-veterinary device as a syringe (100a), or the assembly at the anterior and posterior ends of the cylindrical tube (10) of respective closure caps (106,107) that together with the cylindrical tube (10) form the container (100b) for the processing of biological material in the processing equipment (600),

The two-component piston (20) with the possibility of longitudinal displacement is assembled inside the cylindrical tube (10)

Said piston (20) comprises a rigid rear part (201), of tubular configuration, and a front part (202) made of flexible material coupled to the rear part (201). Said front part (202) includes perimeter sealing lips (203) against the wall of the cylindrical tube and has a valved connector (204) in its central area, accessible through the rear mouth of the tube and suitable for optional connection of a first rod (40) actuating the piston, a second transparent rod (50) provided with an axial channel, or a standard syringe with a suitable connector for the separate extraction of fractions of biological material once processed. The removable rear stop (30) includes lateral tabs (301) to support the fingers and a hole (302) with radial openings (303) for through-mounting and retention in different positions of the first rod (40) drive or the second stem (50) with axial channel.

The first drive rod (40) comprises a front connector (401) that can be mechanically coupled to the valved connector (204) of the flexible component of the piston (20), ergonomic wings (402) for its drive and a row of lateral teeth (403) spaced in the longitudinal direction, which in a first angular position of the first drive rod (40) pass through the radial openings (303) of the rear stop (30) thus allowing the longitudinal displacement of said first drive rod (40) and in a second position interact with the rear stop (30) preventing the longitudinal displacement of said first rod (40)

The second rod (50), with an axial channel, comprises a conical front connector (501) that is an extension of the axial channel that can be attached to the piston valve connection, fixed in this case to the rigid component of the piston by means of bayonet or thread-type mechanical elements and optionally a female Luer Lock cone-type valved rear connector (502) connected by a fine axial conduit (503) for extracting fractions of blood or biological material contained in a cylindrical tube and separated by centrifugation or processing. This second rod (50) also includes ergonomic tabs (504) for manual operation and longitudinal tabs with a row of teeth (505) spaced apart in the longitudinal direction, similar to those of the first drive piston (40), for its locking and unlocking with respect to the rear stop (30) of the cylindrical tube (10).

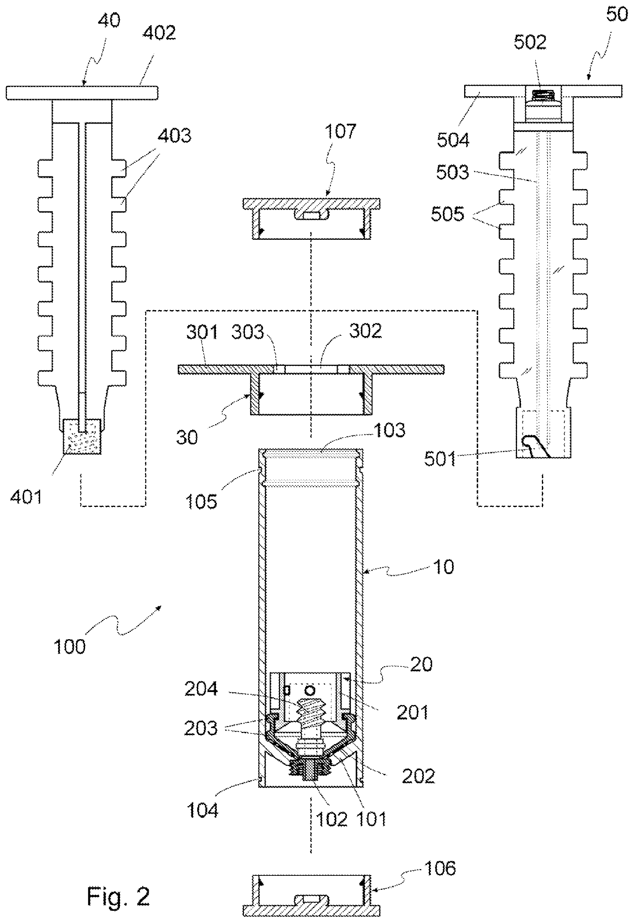

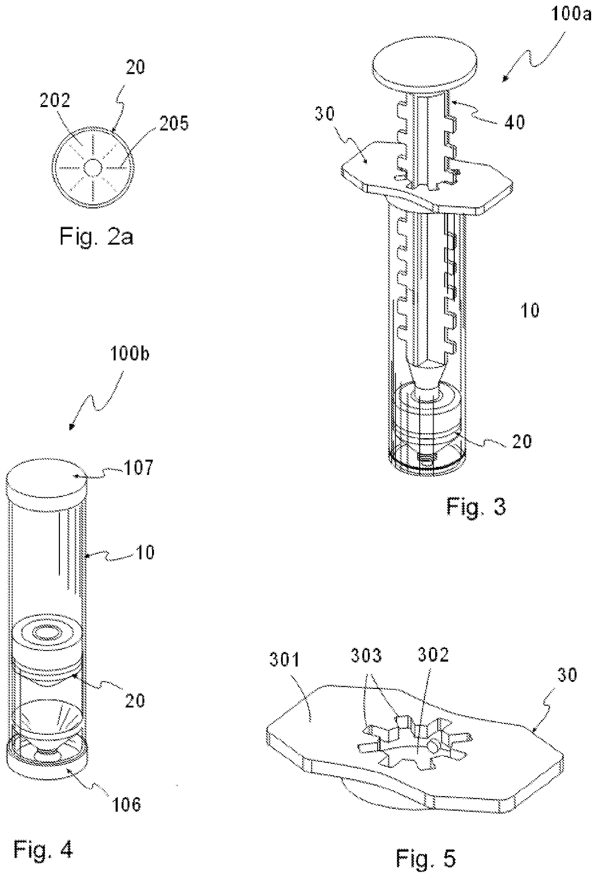

As shown in Figure 2a, the front part (202) of the plunger features radial lines or dots (205) in positive relief.

Figure 3 shows an example of the embodiment of the medical-veterinary device forming a syringe with the cylindrical tube (10), the rear stopper (30) coupled to the tube and the first rod (40) mounted through the rear stopper (30) and mechanically coupled to the plunger (20) -not shown- housed in the cylindrical tube (10).

Figure 4 shows an embodiment of the medical-veterinary device forming a container for the processing of biological material, made up of the tube cylinder (10), the closure caps (106, 107) and the piston housed, in a hidden position, in the cylindrical tube (10).

The perspective of Figure 5 shows an example of the rear stopper (30) with the hole (302) and the radial openings (303) for through mounting and locking the stems (40, 50) in different positions.

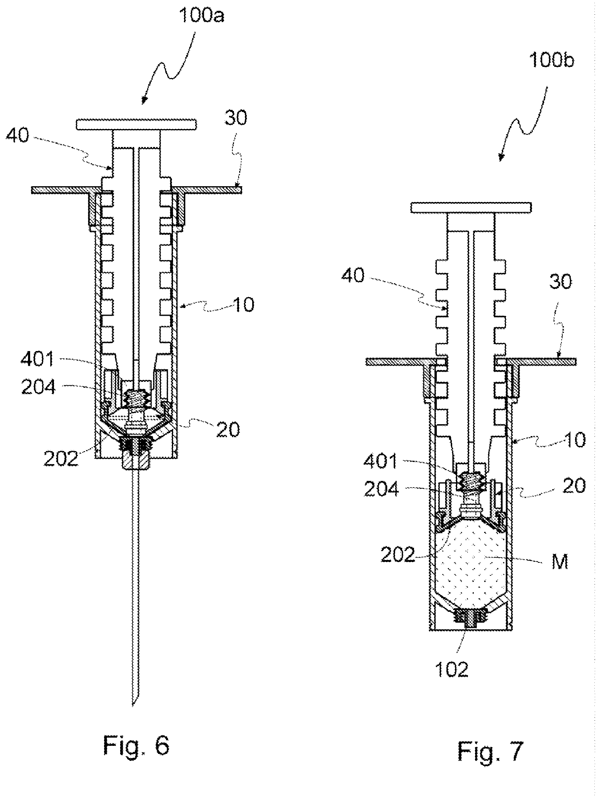

As shown in Figures 1 and 6, in an initial position the front part (202) presents a convex shape, with a tampering which coincides with the front end of the cylindrical tube and is initially kept in contact with the conical front end of the tube by means of the radial lines or dots (205) in relief, avoiding the presence of air between the piston (20) and the conical front end (101) of the cylindrical tube.

In said Figure 6, the medical-veterinary device forms a syringe for the extraction of a biological material, for example, blood, with the first actuation rod (40) being mechanically coupled to the plunger (20) that is in the initial convex position.

Figure 7 shows the syringe of the previous Figure once the first drive rod (40) and consequently plunger (20) have been displaced towards the rear area, the front part (202) changing its morphology from convex to concave and once the extraction of the biological material contained in the device now configured as a syringe has been carried out.

This displacement of the first rod (40), pulling the valved connector (204) of the piston (20) causes the front part of said piston (20) to adopt the concave shape.

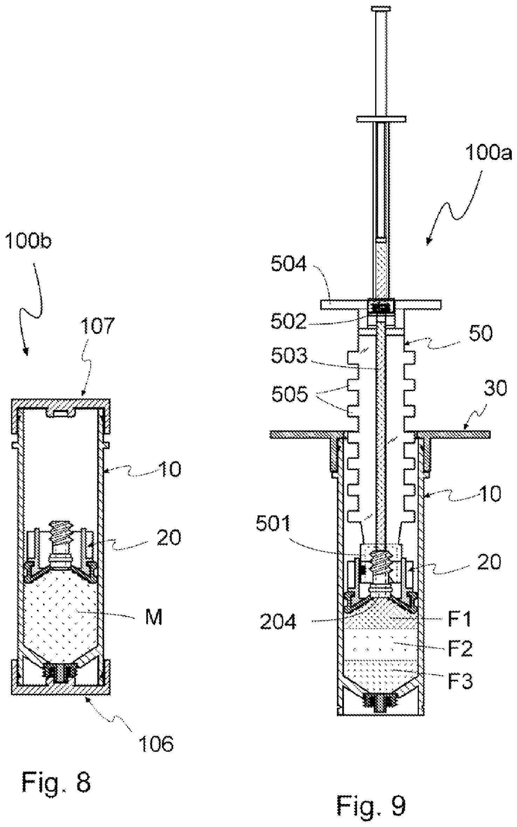

Once the biological material has been extracted, the medical-veterinary device becomes a container, represented in Figure 8, suitable for keeping the biological material (M) inside during its processing in processing equipment (600) as will be described later.

In order to convert the syringe of Figure 7 into the container of Figure 8, it is enough to disassemble the first actuation rod (40) and the rear stop (30) from the cylindrical tube (10), and optionally close the front and rear ends of the cylindrical tube through the corresponding caps (106 and 107).

In case of using the processor in an automated way, it is not essential to use the caps (106, 107), connecting some flexible tubes with fixing elements both to the male Luer Lock cone connection of the cylinder and to the female Luer Lock cone valved connection plunger which, in turn, through peristaltic or other pumps and element/composition sensors of the biological material, will speed up, slow down, stop, or reverse the flow direction through the sensor of the processor to the tank, where the process of the device will collect the separate fractions of interest.

Once the biological material (M) has been processed and its different fractions (for example F1, F2, F3) have been separated, the container of the medical-veterinary device shown in Figure 8 is transformed into a syringe represented in Figure 9 and suitable for the independent extraction of the successive fractions of biological material, simply removing the caps (106, 107) from the cylindrical tube (10), if optionally used, and mounting the rear stop 30 on the same cylindrical tube used for the example of extraction and, in this case, the second stem (50) with axial channel for the extraction of said fractions through its fine axial duct (503) in order of higher to lower fraction.

In Figure 8 said extraction of the fraction (F1) is optionally carried out by connecting a standard syringe (not referenced) to the rear valved connection (502) of the axial channel rod (50) or by means of a syringe directly coupled to the plunger without the second coupled stem.

Optionally, a flexible tube can also be coupled directly to the plunger, provided with suitable connectors to motorize the extraction of the fractions, optionally using peristaltic pumps or any device that allows the fractions obtained to be aspirated in an automated and continuous manner, without the manual use of elements /components such as those described at the beginning of this chapter.

The concave shape of the front part (202) of the piston (20) is especially advantageous during the extraction of the fraction (F1) and all the following fractions of biological material, through the interior of the second rod (50) since the suction is carried out through the most rearward central area of the front part of the piston, thus avoiding the formation of turbulence and, for example, preventing the first fraction (F1) from mixing during its extraction with the next fraction (F2) and so on with all the fractions.

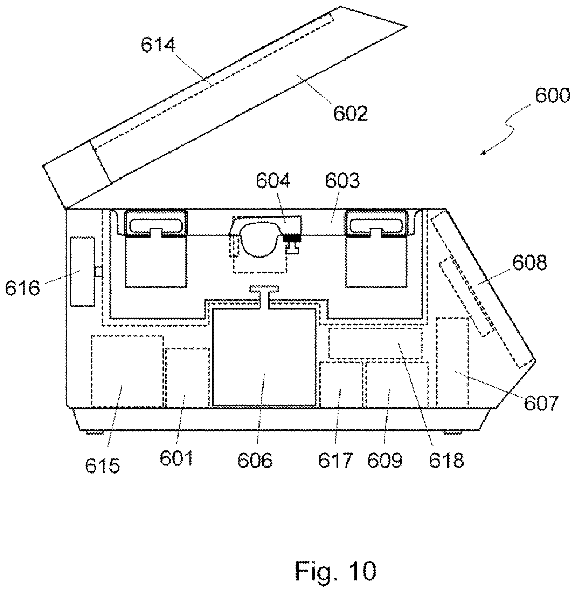

The processing equipment (600) shown in Figure 10 and used for processing the biological material inside the container of the medical-veterinary device described above, comprises an outer casing (601) provided with a lid (602) and housing a turntable (603) driven by a motor (606) for bidirectional rotation connected to a frequency inverter (609) controlled by a control unit (607).

Said cap (602) has some ultraviolet light lamps (614) inside for the disinfection of the interior of the equipment and/or its use as a trigger and trainer of biological processes in the samples (for example, blood or plasma coagulation).

The turntable (603) comprises fixings (604) for holding a container (100b) containing a biological material to be processed and an empty container (100b) for collecting a separate fraction of the biological material.

In this embodiment, the processing equipment also comprises: - a cold or heat generator (616) in order to eventually control the temperature of the sample with different aims such as the reduction of processing times, an increase of biological processes, an optimal maintenance of the samples, etc.; - a transformer (617) for power supply and - an internal or external ultrasonic generator (618).

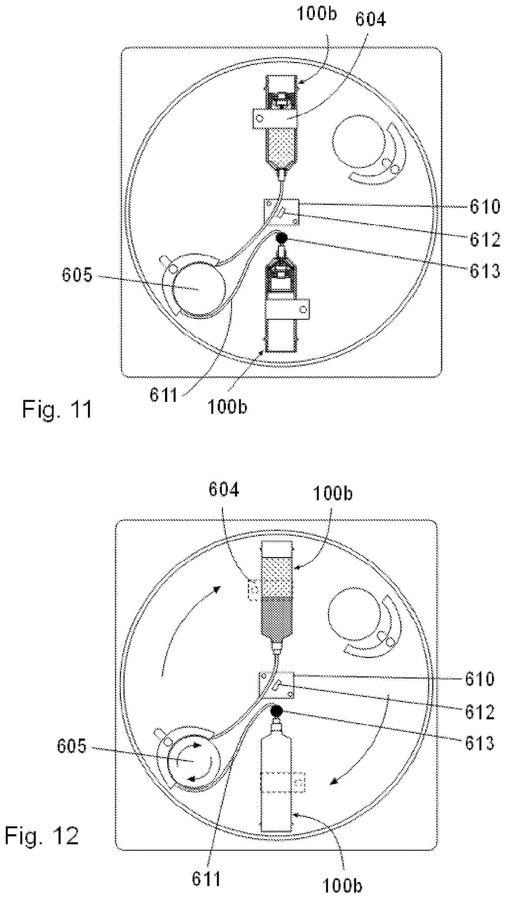

Figures 11 to 13 show a pair of containers (100b) connected by a transparent tube (611), preferably flexible and made of silicone, provided with connectors (not referenced) coupled to the Luer Lock connectors (102 and 204) the respective containers (100b) and a peristaltic pump (605) for the transfer from one container (100b) to another of a fraction of the biological material, separated during a processing cycle, optionally by one connector or by another or both at the same time, depending on the fraction of interest.

The processing equipment (600) comprises a support (610) for holding an intermediate portion of the transparent tube (611) and a laser sensor (612) that detects, for example, the color (also optionally the sensor in the flexible tube line can be a detector, reader, counter and identifier of particles, their size, their number and their geometry/morphology or their biological, chemical or biochemical composition, using optical or digital imaging technology, impedance, laser or other technologies that conform to these readings, counts and identifications), of the final product transferred through the transparent tube (611) and that causes different actions/reactions of the pump such as acceleration, braking, change of direction of rotation and the stoppage of the peristaltic pump (605) when, for example, it detects the change in color of the product, due to the change in the fraction that circulates through said tube, or the activation of other functions or processes such as ultrasound, temperature, agitation or similar.

Said transparent tube (611) comprises an air purge filter (613) to prevent air from being transferred together with the separated fraction of the processed biological material.

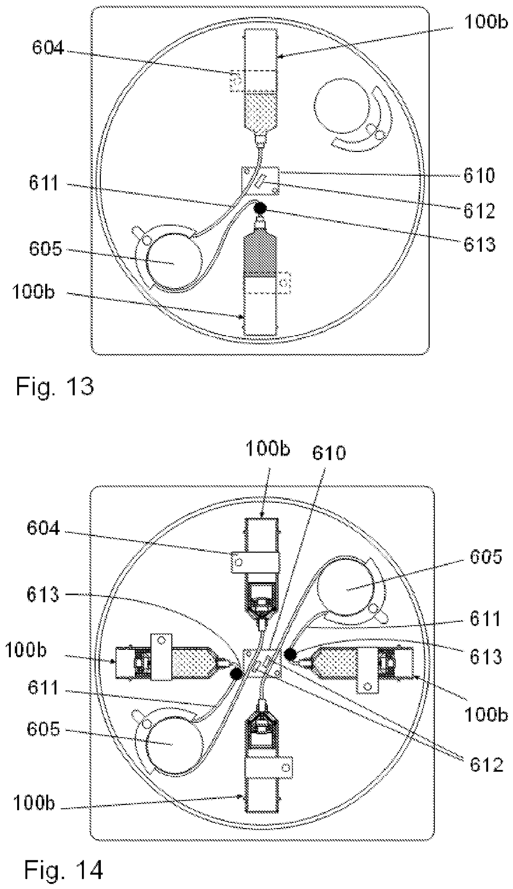

Figure 14 shows a plan view of a variant embodiment of the processing equipment working simultaneously with two pairs of containers (100b).

The connections with the transparent tube (611) can also be made at the rear of the cylindrical tube, particularly connected to the second plunger (204) in order to remove fractions from this part of the device or simultaneously from the front (102) and rear (204) of the device depending on the needs of the user.

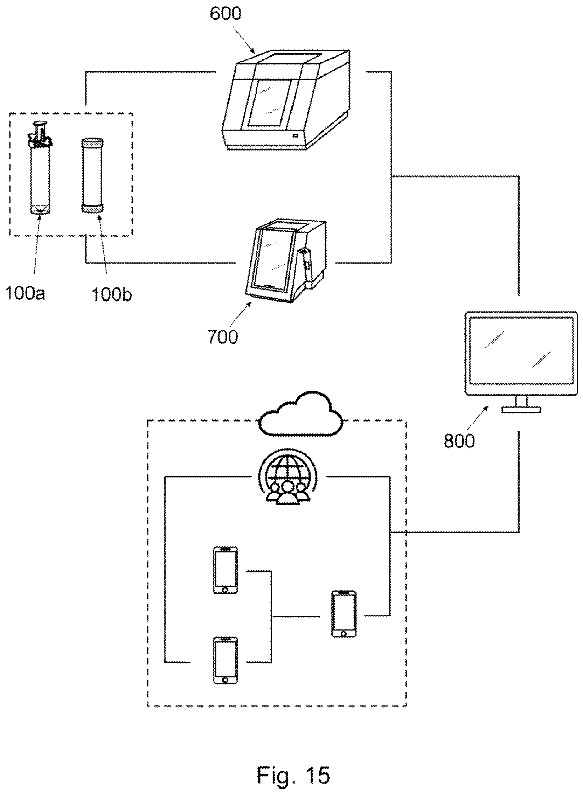

This processing equipment (600) includes a touchpad (608) connected to the control unit (607) and has software that allows it to work independently, without Internet connection, carrying out predefined processing cycles, or to work "on line" with internet connection as shown in Figure 15.

In this embodiment, the processing equipment (600) is connected to a counting or analysis equipment, for example, a hematology analyzer (700) that determines the characteristics of the biological material to be processed and sends it to the processing equipment (600) in such a way that it performs in each case, with each sample from each patient, a customized processing cycle based on the characteristics of the biological material to be processed. Said hematological analyzer (700) can also determine the characteristics of the final product obtained from processing,

In this embodiment, the processing equipment (600) includes a communication module (615) for receiving information related to the characteristics of the product to be processed, sending data related to the final product obtained from processing, and connecting to a computer (800) and to remote devices, an App, or the cloud.

This communication module allows the control and traceability of the tubes/cylindrical tanks through barcodes, QR or RF microchip among others, for control of quality, for analysis of samples and pre- and post-processed biological material by means of particle identifier, their size, their number and their geometry/morphology or biological, chemical or biochemical composition, by means of optical, digital imaging technology, also by impedance, laser or other technologies that adjust to these readings, counts and identifications, as well as to coordinate and execute variable processes of the biological samples in the processor according to readings, for optimization of the final biological product as well as the collection of individual data, protocols and identification and history of the sample and/or patients, for remote control and diagnosis and telemedicine/veterinary/analysis, data export and reading use on any current and future platform (PC, Mac, laptops, phones and smart terminals, in relation to and in use with AI (Artificial Intelligence), loT (Internet of Things), IT (Information Technology), ICT (Information and Communication Technologies), social networks, among others.

Once the nature of the invention has been sufficiently described, as well as a preferred embodiment, it is stated for the appropriate purposes that the materials, shape, size and arrangement of the elements described may be modified, as long as this does not imply an alteration of the essential characteristics of the invention that are claimed below.

## Claims

1. Medical-veterinary device for the extraction and containment of biological material to be processed; wherein said medical-veterinary device (100) comprises interchangeable parts for the formation of a syringe (110a) for extracting biological material, a container (100b) for the extracted biological material to be processed, and a syringe or application system, said parts comprising:
- A transparent cylindrical tube (10), which has a conical front end (101) with a connector (102), and an open rear end (103), both ends (101, 103) being provided with alternative coupling means (104, 105) of removable elements with fixation (106, 107, 30);
- A plunger (20) that can be moved inside the cylindrical tube (10) comprising:
- A rear rigid part (201), with a tubular configuration and a section smaller than the interior of the cylindrical tube (10) and,
- A front part (202) made of flexible material, coupled to the rear part (201), provided with perimeter lips (203) for closing against the cylindrical tube (10) and deformable between: an initial convex position coinciding with and in contact with conical front end (101) of the cylindrical tube (10), and a concave position of use; said front part (202) comprising, in its central area, a valved connector (204) accessible through the rear end (103) of the cylindrical tube (10);
- a first rod (40) for driving the piston (20), provided with a front connector (401) that can be mechanically coupled to the valved connector (204) of the piston (20) and whose displacement causes the change from the initial convex position to the concave position of use of the front part (202) of the piston (20) during the suction of blood or biological material.

2. The medical-veterinary device, according to claim 1, comprising a second stem (50), transparent, with a longitudinal axial channel (50), provided with a front connector (501) and a mechanical fixing that can be attached to the valved connector (204), the rear part (201) of the plunger (20) and a valved rear connector (502), both connectors (501, 502) being connected by the axial channel (503) suitable for extracting fractions of blood or biological material contained in the cylindrical tube (10) and separated by centrifugation or any other processing of interest.

3. The medical-veterinary device, according to any of the previous claims, where the cylindrical tube (10) comprises removable caps (106, 107) for closing the front and rear ends (101, 103) of the cylindrical tube (10) and which together with said cylindrical tube (10) forming a container (100b) that can be attached to a biological fluid processing equipment (600) and obtaining fractions of concentrates by means of centrifugation, agitation, filtration, temperature, ultrasonic sounds, laser, fragmentation, grinding or digestion.

4. The medical-veterinary device, according to any of the previous claims, where the cylindrical tube (10) comprises a removable rear stop (30) provided with: some lateral support tabs (301) and a hole (302) with radial openings (303) for through mounting and retention in different positions of the first and second rod.

5. The medical-veterinary device, according to claim 3 above, wherein the first and second stems (40, 50) comprise: ergonomic wings (402, 504) for its actuation and at least one row of lateral teeth (403, 505) spaced in the longitudinal direction, which in a first angular position of the stem (40, 50) pass through the radial openings (303) of the removable rear stop (30) allowing its longitudinal displacement and in a second angular position of the stem (40, 50) are hooked on the removable stop (30) thus preventing the longitudinal movement of said stem (40, 50).

6. Equipment for processing a biological material contained in the medical-veterinary device of the previous claims; wherein said processing equipment (600) comprises: an outer casing (601) provided with a closing lid (602), a rotating platform (603) driven by a motor (606) for bidirectional rotation, and provided with at least two fixings (604) for the placement of a container (100b) containing the biological material to be processed and an empty container (1 00b), for collecting a separate fraction of the processed biological material, a transparent tube (611), with connectors attachable to the connectors (102) of the respective cylindrical tubes (10) of said containers (100b); and of one or more peristaltic pumps (605) for the transfer of a separate fraction of the biological material from one container (100b) to another through said flexible tube (611), a control unit (607) with specific software in order to carry out predefined processing cycles, or personalized processing cycles based on the characteristics of the biological material to be processed.

7. The processing equipment (600), according to claim 6, comprising at least one support (610) for holding an intermediate portion of the transparent tube (611) and a sensor (612) suitable for detecting the color, optionally the composition, size or morphology of the components of the biological fluid or changes in the product that passes through the transparent tube (611), and that causes changes in speed, deceleration, acceleration, direction rotation and flow or even stopping the peristaltic pump (605) when it detects a change in color or characteristics of the product in circulation.

8. The processing equipment according to any one of claims 6 and 7, wherein the transparent tube (611) comprises an air purge filter (613).

9. The processing equipment according to any one of claims 6 to 8, comprising a touchpad (608) connected to the control unit (607).

10. The processing equipment, according to any one of claims 6 to 9, wherein said processing equipment (600) is connected to an analyzer (700) that determines the characteristics of the biological material to be processed and sends them to the processing equipment (600).

11. The processing equipment, according to any one of claims 6 to 10, comprising a communication module (615) for receiving information relating to the characteristics of the product to be processed, sending data relating to the final product obtained from processing and the connection with a computer (800) and with remote devices, an App, or the cloud.

12. The processing equipment, according to any one of claims 6 to 11, in which the lid (602) has ultraviolet light lamps (614) inside for disinfection inside the processing equipment.

13. The processing equipment, according to any one of claims 6 to 12, comprising a cold or heat generator (616) suitable for controlling the temperature of the samples to be processed.

14. The processing equipment according to any one of claims 6 to 13, comprising a power supply transformer (617) .

15. The processing equipment, according to any one of claims 6 to 14, has an ultrasound generation module (618) inside it, suitable for subjecting the samples to micronization and emulsification processes.
